# EUROPEAN PATENT APPLICATION

(11) **EP 3 904 494 A1**
(43) Date of publication of application: **03.11.2021**
(21) Application number: 20315231.9
(22) Date of filing: 30.04.2020
(51) Int. Cl.: C12M 1/00, C12M 1/36, C12M 1/34

(54) **AERATION DEVICE FOR A BIOPROCESSING INSTALLATION**

(71) Applicant: Sartorius Stedim Biotech GmbH, 37079 Göttingen (DE)
(72) Inventor: Gärtner, Sascha, 36205 Sontra (DE); Husemann, Ute, 37079 Göttingen (DE); Schubert, Jan-Eike, 37077 Göttingen (DE); Höhl, Clemens, 37120 Bovenden (DE); Böttcher, Lars, 34212 Melsungen (DE); Loiselet, Yanis, 13600 Ceyreste (FR); Leupold, Marco, 37075 Göttingen (DE); Scheibe, Oliver, 36037 Fulda (DE); Wollschmidt, Regina, 37170 Uslar (DE); Kues, Dominic, 37079 Göttingen (DE)
(74) Representative: Casalonga

(57) **Abstract**

The invention relates to an aeration device for a bioprocessing installation (1), in particular a bioreactor (2), comprising a housing (9), wherein the housing (9) comprises one or more aeration channels (10a, 10b) in its interior, wherein the housing (9) further comprises one or more gas inlet ports (11), via each of which a gas can be introduced into at least one aeration channel (10a, 10b) of the housing (9), and wherein the housing (9) further comprises a plurality of gas outlet ports (12a, 12b), via each of which the gas can be discharged from the respective aeration channel (10a, 10b) to the outside of the aeration device (8). It is proposed that the aeration device (8) is configurable with respect to a predetermined discharge of gas via the gas outlet ports (12a, 12b).

## Description

The invention relates to an aeration device for a bioprocessing installation, in particular a bioreactor, according to the general part of claims 1, 29, 30, 31 and 32, and to a bioprocessing installation with such an aeration device according to claim 33.

A bioprocessing installation is generally understood to be an apparatus with which biotechnological processes, e.g. biopharmaceutical processes, can be carried out or supported. Bioreactors, in which microorganisms or mammalian cells are cultivated under given conditions, are only mentioned here as examples. Another example of bioprocessing installations are process mixing systems that can be used to mix liquid biological mediums such as suspensions, solutions, emulsions or the like, that are used in biotechnological processes. Such bioprocessing installations regularly have a container, in which a liquid biological medium consisting of the substances intended for the biotechnological process, for example microorganisms or tissue cells on the one hand, and a corresponding nutrient medium on the other hand, is accommodated in order to be able to carry out the respective biotechnological process step, for example fermentation or cultivation and/or mixing. The product, in case of a bioreactor for example the fermentation broth, is then usually further treated in a so-called downstream process to obtain a product from the cells or the culture supernatant.

A container for such a bioprocessing installation can be a dimensionally stable vessel on the one hand, but on the other hand also a flexible, i.e. bendable and slack, bag, also known as bioprocess bag.

Provision of an oxygen supply is one key factor in cellular metabolic processes. Although tissue cell cultures consume substantially less oxygen than bacteria and yeast cultures, ensuring an efficient supply is the greatest challenge facing the operation of a bioprocessing installation, in particular bioreactor. In addition to supplying the cells with oxygen, i.a. the concentration of dissolved carbon dioxide also plays a part as a controlled variable.

There are two conventional aeration methods: Aerating the headspace of the bioreactor and direct injection of gases through aeration devices such as aeration rings or discs. For the latter purpose, use is made not only of aeration rings or discs ("ring spargers") comprising gas outlet ports having a discharge opening with a diameter larger than 0,2 mm, for example 0,8 mm, but also of "mico-spargers", which are aeration devices with gas outlet ports having a discharge opening with a smaller diameter, for example 0,15 mm. Both types of aeration devices have specific advantages and drawbacks. It is therefore also known to combine both types of gas outlet ports in a common housing ("combined sparger").

The aeration device (US 10,427,112 B2), which is the starting point of the invention, comprises a housing with two aeration channels in its interior. The housing further comprises two gas inlet ports via each of which a gas can be introduced into an assigned aeration channel of the housing. Moreover, the housing comprises a plurality of gas outlet ports provided in the form of a first type of gas outlet ports having larger openings and a second type of gas outlet ports having smaller openings. Each type of gas outlet ports is assigned to a respective one of the aeration channels.

The known aeration device can be used as ring sparger, making use of the larger openings, and as microsparger, making use of the smaller openings. This aeration device is already very advantageous, as it can be uses for two different applications. Nevertheless, an aeration device would be desirable which could still be used for a wider range of applications. Also an aeration device would be desirable which as much as possible prevents an aeration device from unintentional filling up with the liquid biological medium and/or which prevents unintentional clogging (blocking of the discharge openings).

It is therefore an object of the present invention to provide an aeration device for a bioprocessing installation, in particular a bioreactor, which can be used for a wider range of applications.

The above noted problem is solved by an aeration device for a bioprocessing installation, in particular a bioreactor, with the features of the general part of claim 1 by the features of the characterizing part of claim 1.

The idea underlying the invention is to have an aeration device which can be adapted to different situations. It is a basic consideration that by manually or automatically configurating an aeration device of a bioprocessing installation, in particular a bioreactor, certain characteristics of the gas discharged from the aeration device into for example a liquid biological medium can be influenced. Such characteristics are, just as an example, the discharge velocity of the gas, the discharge locations of the gas, the discharge direction of the gas, the gas composition and/or the gas bubble size and/or gas bubble shape at the opening of the respective gas outlet port. By influencing certain characteristics of the gas, the behavior of the aeration device can be adapted to different requirements, especially to different biotechnological processes and/or to different stages within a biotechnological process. Moreover, such an aeration device can prevent unintentional filling up with liquid biological medium and/or unintentional clogging as will be explained later.

In detail, it is proposed that the aeration device is configurable with respect to a predetermined discharge of gas via the gas outlet ports. The device is therefore designed to allow certain changes to it, manually or automatically, in order to reach and/or maintain a predetermined discharge of gas via the gas outlet ports.

Claim 2 defines different types of gas outlet ports of the housing of the aeration device. The gas outlet ports of a first type have openings with the typical diameter or cross-sectional area of openings of a standard ring sparger. The gas outlet ports of a second type have openings with the typical diameter or cross-sectional area of openings of a standard microsparger. The proposed aeration device may comprise only one of the first and second type of gas outlet ports or both types of gas outlet ports.

Claims 3 to 20 relate to an embodiment of an aeration device which is configurable by adjustment using an adjusting mechanism, whereas claims 21 to 28 relate to an embodiment of an aeration device which is configurable by replacement of one or more housing parts of the aeration device housing. It is to be noted that both embodiments may also be combined in one aeration device.

Regarding the embodiment of an aeration device which is configurable by adjustment using an adjusting mechanism, according to claim 4 a movable closing element for releasing and for blocking a fluid communication between one or more aeration channels and the outside of the aeration device may be provided. Such a closing element may form an upper cover of the housing (claim 5). Claims 5 to 11 specify particularly preferred designs and functionalities of the clothing element.

In another embodiment, according to claim 12 additionally or alternatively a movable closing element for releasing and for blocking a fluid communication of one or more gas inlet ports to one or more aeration channels may be provided. Such a closing element may form a lower cover of the housing (claim 13). Claims 13 to 19 specify particularly preferred designs and functionalities of the clothing element.

According to the preferred embodiment in claim 20, the adjusting mechanism is configured to be controlled by an electronic control unit of the bioprocessing installation. This makes it particularly easy to adapt the aeration device to different biotechnological processes, or to adapt the aeration device to different stages and/or to changing conditions in a biotechnological process, even during the course of the biotechnological process.

Regarding the embodiment of an aeration device which is configurable by replacement of one or more housing parts of the aeration device housing, according to claim 21 housing parts, which have properties influencing the discharge of gas via the gas outlet ports, are mutually replaceable.

According to claim 22 at least one housing part may be detachably connected to the remainder of the housing by a positive connection and/or non-positive connection. Claims 23 to 28 specify particularly preferred designs and functionalities of the mutually replaceable housing parts and the respective aeration device.

An independent second teaching according to claim 29 is directed to an aeration device for a bioprocessing installation, in particular the aeration device according to the first teaching. In that aeration device the surface on the inside of one or more gas outlet ports of the housing and/or of at least a section of one or more aeration channels of the housing is provided with a coating or insert. The coating or insert is preferably made of a hydrophobic material, more preferably a silicone material. All explanations given for the aeration device according to the first teaching are fully applicable to this teaching.

An independent third teaching according to claim 30 is directed to an aeration device for a bioprocessing installation, in particular the aeration device according to the first or second teaching. In that aeration device for one or more aeration channels of the housing a membrane and/or a filter material is arranged between the respective aeration channel and one or more gas outlet ports of the housing, such that the gas can be discharged from the respective aeration channel through the membrane and/or filter material to the outside of the aeration device. Additionally or alternatively, for one or more aeration channels of the housing a membrane and/or a filter material is arranged inside one or more gas outlet ports of the housing, such that the gas can be discharged from the respective aeration channel through the membrane and/or filter material to the outside of the aeration device. All explanations given for the aeration device according to the first and second teaching are fully applicable to this teaching.

An independent fourth teaching according to claim 31 is directed to an aeration device for a bioprocessing installation, in particular the aeration device according to one of the first to third teaching. In that aeration device one or more aeration channels of the housing are bordered at the top by a membrane made of a flexible material, preferably a silicone material, wherein the membrane forms one or more gas outlet ports of the housing. Preferably, due to the flexibility of the material of the membrane, the size of the cross-sectional area of the gas outlet ports formed by the membrane changes depending on the gas pressure in the respective aeration channel. All explanations given for the aeration device according to the first to third teaching are fully applicable to this teaching.

An independent fifth teaching according to claim 32 is directed to an aeration device for a bioprocessing installation, in particular the aeration device according to one of the first to fourth teaching. In that aeration device at least some of the gas outlet ports of a respective one of the aeration channels are non-uniform, or at least one group of gas outlet ports of a respective one of the aeration channels is non-uniform. Preferably the size of the cross-sectional area of the gas outlet ports of at least one of the aeration channels varies along the aeration channel. Additionally or alternatively, the shape of the cross-sectional area of at least some of the gas outlet ports of at least one of the aeration channels may not be circular. All explanations given for the aeration device according to the first to fourth teaching are fully applicable to this teaching.

An independent sixth teaching according to claim 33 is directed to a bioprocessing installation, in particular a bioreactor, comprising a container, preferably a dimensionally stable vessel or flexible bag, in which a liquid biological medium consisting of the substances intended for a biotechnological process can be accommodated, further comprising a proposed aeration device according to one of the first to fifth teaching. All explanations given for the aeration device according to the first to fifth teaching are fully applicable to this teaching.

In the following, embodiments of the invention are explained with respect to the drawing. In the drawing
- Fig. 1: shows a proposed bioprocessing installation with a proposed aeration device,
- Fig. 2: shows a top view and a sectional view of a first exemplary embodiment of the proposed aeration device,
- Fig. 3: shows a top view and sectional views of a second exemplary embodiment of the proposed aeration device,
- Fig. 4: shows a top view and a sectional view of a third exemplary embodiment of a proposed aeration device,
- Fig. 5: shows a detail of a proposed aeration device a) according to a first example, b) according to a second example, c) according to a third example and d) according to a fourth example, and
- Fig. 6: shows a top view and a sectional view of a fourth exemplary embodiment of the proposed aeration device.

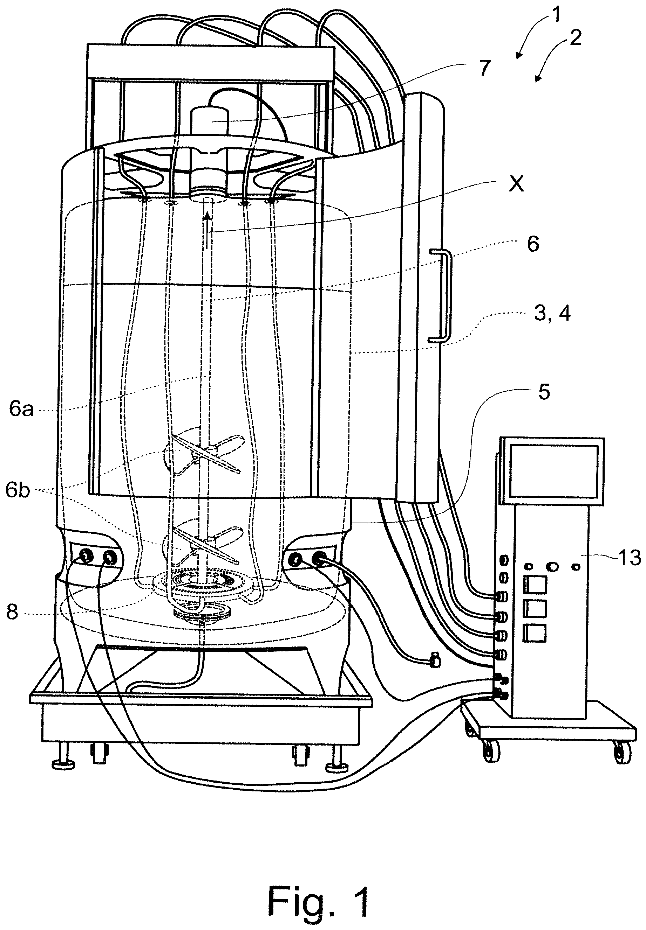

An exemplary embodiment of a proposed bioprocessing installation 1 in the form of a bioreactor 2 is schematically shown in Fig. 1. The bioreactor 2 comprises a container 3, in particular a single-use container, in which a liquid biological medium, in this case a biological reaction medium, consisting of the substances intended for a biotechnological process can be accommodated. The biological reaction medium comprises in particular microorganisms such as bacteria, yeasts, viruses or the like or tissue cells such as mammalian cells, plant cells or the like, and a nutrient medium. The container 3 is here designed as a bioprocess bag 4, i.e. as a container with walls which are flexible at least in sections. In principal, the container 3 can also be designed as a dimensionally stable vessel in an alternative form (not shown). The container 3 may in principal also be a multi-use container instead of a single-use container.

In order to form a microbial or cellular product, the microorganisms or tissue cells are cultivated in the container 3 under specified conditions. In addition to the microorganisms or tissue cells on the one hand and the nutrient medium on the other hand, the container 3 allows further liquid or gaseous media, such as oxygen, pH correcting agent or the like, to be added, and the fermentation broth produced by the cultivation and any gases which may have collected in the headspace of the container 3 to be discharged. This discharged fermentation broth can then be fed to, among other things, a filter or centrifuge to obtain the final product.

Another example of a proposed bioprocessing installation 1 not shown here is a process mixing system that is used for mixing suspensions, solutions or emulsions that can be used in biotechnological processes, for example in a downstream or upstream process.

The proposed bioprocessing installation 1, here in the form of the bioreactor 2, also comprises a holder 5, here in the form of a stainless steel housing, in which the container 3 is installed. In the installed state, the container 3 extends in axial direction X, here in the vertical or gravitational direction, from an upper container end to a lower container end.

Here and preferably, the bioprocessing installation 1 comprises a stirrer 6, which is installed inside of the container 3. The stirrer 6 has here and preferably a stirrer shaft 6a extending in axial direction X from the upper container end towards the lower container end. The stirrer shaft 6a is equipped with at least one stirring element 6b, here in the form of a propeller stirrer. Here and preferably, two stirring elements 6b are provided at the stirrer shaft 6a. The stirrer 6 is rotatably driven by a drive motor 7 installed outside of the container 3 at the holder 5. The stirrer 6 here is used for mixing the biological medium during cultivation.

As also shown in Fig. 1, the bioprocessing installation 1 further comprises an aeration device 8, which serves for the supply of one or more gases to the biological medium, e.g. oxygen, carbon dioxide, nitrogen, air or the like.

The aeration device 8 is located here and preferably below the at least one stirring element 6b. However, it is also conceivable to locate the aeration device 8 or a further aeration device 8 between two stirring elements 6b. Moreover, it is also conceivable to locate the aeration device 8 or a further aeration device 8 above the at least one stirring element 6b. The aeration device 8 or a further aeration device 8 may also be located at the bottom of the container 3 and/or the aeration device 8 or a further aeration device 8 may be located at a side wall of the container 3. Here and preferably, the aeration device 8 is arranged around the stirrer shaft 6a and coaxially thereto. However, the aeration device 8 may also be arranged with an offset to the stirrer shaft 6a. Moreover, the aeration device 8 can be oriented at different angles relative to the axial direction X within the container 3 and does not necessarily have to be oriented horizontally, as in the case shown in Fig. 1.

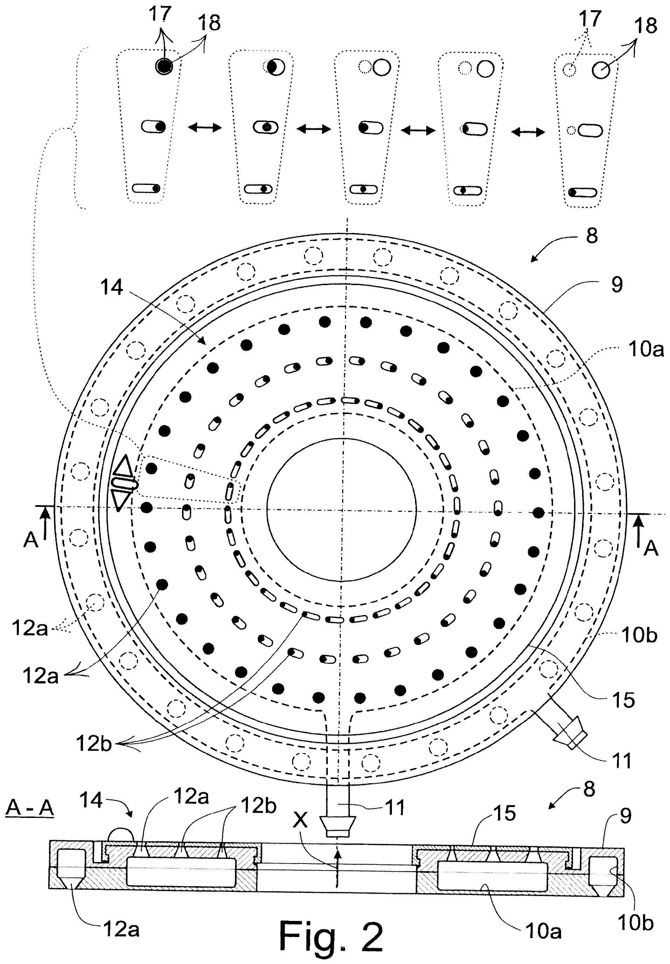

As can be seen in Figs. 2 to 6, the proposed aeration device 8 comprises a housing 9. The housing 9 comprises one or more aeration channels 10a, 10b in its interior. Here and preferably, the housing comprises a top and a bottom, which, when mounted together, form the aeration channels 10a, 10b.

Furthermore, the housing 9 comprises one or more gas inlet ports 11, via each of which a gas can be introduced into at least one aeration channel 10a, 10b of the housing 9. As shown in Fig. 1, in the mounted and ready-to-operate condition each gas inlet port 11 is in fluid communication with a respective gas source via a respective gas supply line. Accordingly, the gas inlet ports 11 each are in fluid communication with at least one aeration channel 10a, 10b of the housing 9 and/or can be brought into fluid communication with at least one aeration channel 10a, 10b of the housing 9.

Furthermore, the housing 9 comprises a plurality of gas outlet ports 12a, 12b, via each of which the gas can be discharged from the respective aeration channel 10a, 10b to the outside of the aeration device 8 and in particular into the liquid biological medium inside of the container 3. Accordingly, the respective aeration channel 10a, 10b of the housing 9 is or can be brought in fluid communication with the outside of the aeration device 8 and in particular with the liquid biological medium inside of the container 3 via the gas outlet ports 12a, 12b assigned to the respective aeration channel 10a, 10b.

The gas outlet ports 12a, 12b assigned to a respective aeration channel 10a, 10b may have different shapes in longitudinal section, e.g. a cylindrical, conical or funnel-shaped shape. The sidewalls of the gas outlet ports 12a, 12b, e.g. the conical gas outlet ports 12a, 12b, may be oriented with an angle between 15° and 40° to the middle axis in flowing direction of the gas outlet port 12a, 12b.

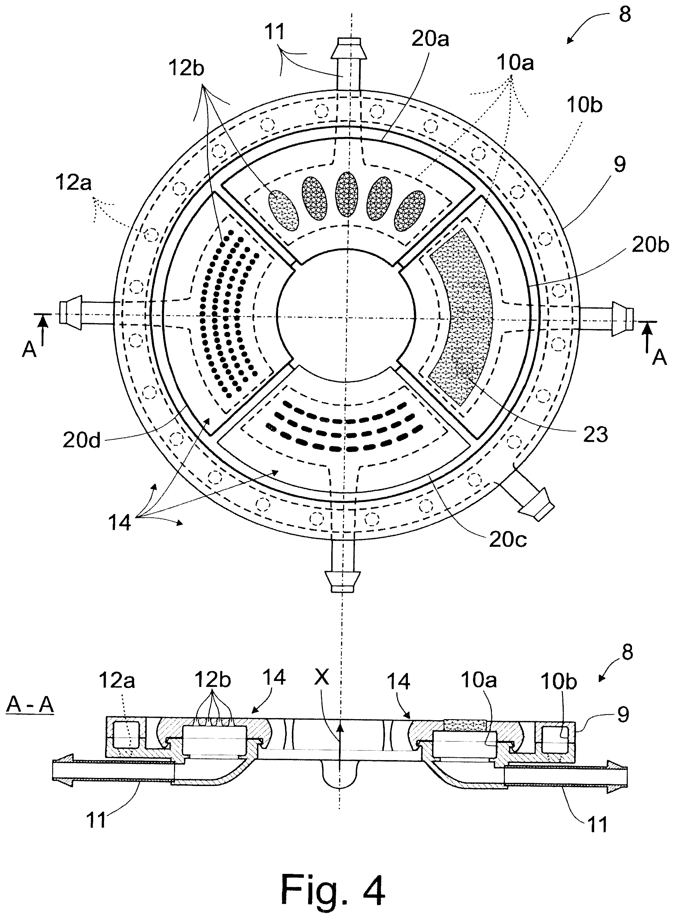

In a plan view parallel to the rising direction of the gas bubbles, according to one variant the gas outlet ports 12a, 12b of one or more aeration channels 10a, 10b are arranged in at least one row, in particular in at least one row running circumferentially over a circumferential section (Fig. 4) or the entire circumference (Figs. 2, 3, 6), along the aeration channel 10a, 10b. One or more rows of gas outlet ports 12a, 12b may be provided per aeration channel 10a, 10b, the several rows being arranged in particular next to each other and/or concentrically to each other (Figs. 2, 4). Also the rows of gas outlet ports 12a, 12b of different aeration channels 10a, 10b are in particular arranged next to each other and/or concentrically to each other (Figs. 2, 3, 4, 6). According to another variant the gas outlet ports 12a, 12b of one or more aeration channels 10a, 10b are arranged in the form of areas covered by a perforated membrane and/or filter material, e.g. a sintered material, which will be described later in more detail (Fig. 4).

One or more, in particular all, aeration channels 10a, 10b and/or rows of gas outlet ports 12a, 12b may be arranged, in the rising direction of the gas bubbles, with the stirring element or elements 6b aligned and/or circumferentially outside.

Each row may comprise one or more, e.g. up to 25 or even more gas outlet ports 12a, 12b.

It is now essential that the aeration device 8 is configurable with respect to a predetermined discharge of gas via the gas outlet ports 12a, 12b. This means that certain characteristics of the aeration device 8 can be changed, manually or automatically, in order to reach and/or maintain a predetermined discharge of gas via the gas outlet ports 12a, 12b.

The term "predetermined discharge" means that the discharge is intended for a specific biotechnological process and/or stage within a biotechnological process, i.e. a discharge which is necessary to allow the biotechnological process and/or stage to run as intended.

As indicated before, the configuration of the aeration device 8 may be carried out manually or automatically.

"Manually" means that a user changes the characteristics of the aeration device 8 by user intervention.

"Automatically" means that the changes of the characteristics of the aeration device 8 are effected, without user intervention, by an electronic control unit 13 of the bioprocessing installation 1, which control unit 13 is provided as shown in Fig. 1 for at least partial control of the bioprocessing installation 1.

The proposed aeration device 8 may comprise different types of gas outlet ports 12a, 12b. For example, gas outlet ports 12a can be provided in the form of a first type of gas outlet ports 12a. Said first type consists of gas outlet ports 12a having an discharge opening 17 with a diameter larger than 0,2 mm, preferably of 0,25 to 4 mm, more preferably of 0,5 to 1,0 mm, and/or a cross-sectional area larger than 0,03 mm², preferably of 0,05 to 12,0 mm², more preferably of 0,2 to 0,8 mm². Additionally or alternatively, gas outlet ports 12b can be provided in the form of a second type of gas outlet ports 12b. Said second type consists of gas outlet ports 12b having an discharge opening 17 with a diameter of at most 0,2 mm, preferably of 0,005 to 0,2 mm, more preferably of 0,02 to 0,18 mm, and/or a cross-sectional area of at most 0,03 mm², preferably of 0,00002 to 0,03 mm², more preferably of 0,0003 to 0,025 mm². According to the exemplary embodiments shown in Figs. 2 to 6, both types of gas outlet ports 12a, 12b are provided, although it is also conceivable that only the first type or only the second type is provided.

Fig. 2 shows an aeration device 8 which has, on the one hand, downward facing gas outlet ports 12a of the first type and, on the other hand, upward facing gas outlet ports 12a, 12b of both the first type and the second type. Facing upward and facing downward means that the respective openings face upward or downward, i.e. in or against the axial direction. The upward facing gas outlet ports 12a, 12b are here all assigned to a common aeration channel 10a, i.e. gas introduced into the aeration device 8 via a respective gas inlet port 11, which is in fluid communication or can be brought into fluid communication with this aeration channel 10a, can be directed through the common aeration channel 10a into the gas outlet ports 12a, 12b and from there to the outside of the aeration device 8. The gas is discharged here at the top of the aeration device 8. This aeration channel 10a and in particular these upward facing gas outlet ports 12a, 12b are used here to configure the aeration device 8.

The downward facing gas outlet ports 12a are assigned to a separate aeration channel 10b, which is positioned concentrically to and here radially outside of the aeration channel 10a, whereby gas introduced into the aeration device 8 via a gas inlet port 11, which is here in permanent fluid communication with this aeration channel 10b, can be directed through the aeration channel 10b into the gas outlet ports 12a of this aeration channel 10b and from there to the outside of the aeration device 8. The gas is discharged here at the bottom of the aeration device 8.

It should be noted that, in addition or alternatively to the upward facing and/or to the downward facing gas outlet ports 12a, 12b, there also may be lateral gas outlet ports 12a, 12b, i.e. with openings facing to the side (in the radial direction), which may be of the first and/or second type, these gas outlet ports 12a, 12b in an optional embodiment also being used to configure the aeration device 8.

Fig. 2 shows an exemplary embodiment, in which the aeration device 8 has an adjusting mechanism 14, here a manually operable adjusting mechanism 14. Instead, also an automatically operable adjusting mechanism 14 may be provided. Furthermore, the aeration device 8 is configurable with respect to a predetermined discharge of gas via the gas outlet ports 12a, 12b in that, by the adjusting mechanism 14, the discharge of gas via the gas outlet ports 12a, 12b is adjustable.

The term "adjustable" means, that for a specific biotechnological process and/or specific stage within a biotechnological process, it is possible, to set a specific state of the gas discharge once, here manually, and/or to adjust it repeatedly during the biotechnological process and/or stage by control (open-loop-control) or by regulation (closed-loop-control), the latter not being shown here.

Setting and/or repeatedly adjusting the state of the gas discharge allows to reach and/or maintain specific conditions in the biotechnological process and/or stage, e.g. to reach and/or maintain a specific gas composition or distribution and/or a specific cell growth etc.

The term "setting a specific state" means that the specific state is established with a choice between several states. In particular, it is possible to, e.g. manually, choose between three or more selectable states or between any number of selectable states. Thereby, a specific discharge of gas can be individually selected depending on the gas discharge requirements of the biotechnological process and/or stage.

As already indicated before, "manually" means that the adjusting mechanism 14 is operated by user intervention, e.g. a closing element 15, 16 such as a slider or flap is manually moved by a user.

The term "automatically" means that the adjusting mechanism 14 is operated, without user intervention, by an electronic control unit 13 of the bioprocessing installation 1, the electronic control unit 13 being provided for at least partial control of the bioprocessing installation 1. For example, an actuator assigned to the adjusting mechanism 14 is operated based on control signals generated by the electric control unit 13, and moves a closing element 15, 16 such as a slider or flap.

In the exemplary embodiment shown in Fig. 2, the adjusting mechanism 14 comprises a movable closing element 15 for releasing and for blocking, at least partly, especially completely, a fluid communication of one or more aeration channels 10a, 10b of the housing 9 to the outside of the aeration device 8.

A "movable" closing element 15 means that the respective closing element 15 is movable relative to the remainder of the housing 9, in particular movable relative to the aeration channel or channels 10a, 10b and/or gas outlet ports 12a, 12b.

The term "for releasing and for blocking" means that in one adjusting position of the closing element 15, when releasing a fluid communication, it allows a fluid communication of one or more aeration channels 10a of the housing 9 to the outside of the aeration device 8, and in another adjusting position of the closing element 15, when blocking a fluid communication, it does not allow a fluid communication of one or more aeration channels 10a of the housing 9 to the outside of the aeration device 8. In particular, the closing element 15 may be adjusted in such a way as to allow complete blocking and/or complete release and/or, in one or more between positions, partial release.

By means of said adjusting mechanism 14 certain characteristics of the discharged gas can be set or repeatedly adjusted, for example characteristics as the discharge velocity of the gas, the discharge locations of the gas (the locations of the discharge openings 17 of the gas outlet ports 12a, 12b in the housing 9), the discharge direction of the gas (discharge from the top, bottom and/or lateral side of the housing 9), the gas composition and/or the gas bubble size and/or gas bubble shape of the discharged gas at the respective discharge opening 17.

In particular, such a closing element 15 prevents the housing 9 and respective gas supply lines connecting a gas source with the respective gas inlet port 11 from filling up with the liquid biological medium if no gas exits through the discharge openings 17 of the gas outlet ports 12a, 12b. The closing element 15 therefore preferably blocks the fluid communication of the respective aeration channel or channels 10a of the housing 9 to the outside of the aeration device 8 until the beginning of an aeration sequence in a biotechnological process, and then releases the fluid communication until the end of the aeration sequence. This is preferably done automatically via the control unit 13. In this context it is also emphasized, that also aligning discharge openings 17, here of the aeration channel 10b, downwards prevents the housing 9 and respective gas supply lines from filling up.

As shown in Fig. 2, the closing element 15 may form an upper cover of the housing 9 and/or may be arranged, in relation to the flow direction of the gas, downstream of the respective one or more aeration channels 10a and/or gas outlet ports 12a, 12b.

The term "upper" means that in the intended installed use state the closing element 15 or cover is arranged on the side of the housing 9 facing in the rising direction of the gas bubbles, that is in the vertical direction and parallel to direction of gravity.

The "respective" one or more aeration channels 10a are those aeration channels 10a, the fluid communication of which to the outside of the aeration device 8 can be blocked by the closing element 15. Accordingly, the "respective" gas outlet ports 12a, 12b are those gas outlet ports 12a, 12b, the fluid communication through which to the outside of the aeration device 8 can be blocked by the closing element 15.

Additionally or alternatively, and as also shown in Fig. 2, the closing element 15 may be a rotary slider or, not shown here, a linear slider. Also a closing element in form of a flap is conceivable.

The closing element 15 here and preferably is movable around the geometrical axis X which runs parallel to the rising direction of the gas bubbles. It is also conceivable, in different embodiments (not shown), that the closing element 15 is movable along the geometrical axis X which runs parallel to the rising direction of the gas bubbles, and/or that the closing element 15 is movable around or along a geometrical axis which runs transversely to the rising direction of the gas bubbles.

Here and preferably, the closing element 15 shown in Fig. 2 is disk shaped.

As can be seen in Fig. 2, here and preferably the closing element 15 comprises one or more closing element openings 18 which, for releasing, at least partly, especially completely, the fluid communication of one or more aeration channels 10a of the housing 9 to the outside of the aeration device 8, can each be brought at least in partial, especially in complete, overlap with at least one discharge opening 17. Additionally or alternatively, the one or more closing element openings 18, for completely blocking the fluid communication of one or more aeration channels 10a of the housing 9 to the outside of the aeration device 8, can each be positioned completely offset to at least one discharge opening 17.

Here and preferably, the closing element 15 is arranged to release and to block, at least partly, especially completely, a fluid communication of one or more, but not all, aeration channels 10a, 10b of the housing 9 to the outside of the aeration device 8, and to not affect a fluid communication of one or more other aeration channels 10b, here one single other aeration channel 10b, of the housing 9 to the outside of the aeration device 8.

According to the exemplary embodiment in Fig. 2 it is provided that the one or more aeration channels 10a, the fluid communication of which to the outside of the aeration device 8 can be released and blocked by the closing element 15, are in fluid communication with and/or can be brought into fluid communication with both the first type and the second type of gas outlet ports 12a, 12b.

However, it is also conceivable, in a different embodiment (not shown), that the one or more aeration channels 10a, the fluid communication of which to the outside of the aeration device 8 can be released and blocked by the closing element 15, are in fluid communication with and/or can be brought into fluid communication with only one type of gas outlet ports 12a, 12b, preferably only the second type of gas outlet ports 12b.

Here and preferably, the gas outlet ports 12a, 12b of the one or more aeration channels 10a, the fluid communication of which to the outside of the aeration device 8 can be released and blocked by the closing element 15, point in the rising direction of the gas bubbles. Additionally or alternatively, said gas outlet ports 12a, 12b or some of said gas outlet ports 12a, 12b may point transverse to and/or against the rising direction of the gas bubbles.

Additionally or alternatively, and as also shown in Fig. 2, the one or more other aeration channels 10b, here the single other aeration channel 10b, the fluid communication of which to the outside of the aeration device 8 cannot be affected by the closing element 15, are/is in permanent fluid communication with only one type of gas outlet ports 12a, 12b, here and preferably only the first type of gas outlet ports 12a.

Here and preferably, the gas outlet ports 12a assigned to the one or more other aeration channels 10b, here the single other aeration channel 10b, point in a different, in particular opposite, direction than the gas outlet ports 12a, 12b assigned to the aeration channel or channels 10a, the fluid communication of which to the outside of the aeration device 8 can be released and blocked by the closing element 15. More preferably, these gas outlet ports 12a assigned to the one or more other aeration channels 10b, here the single other aeration channel 10b, point against the rising direction of the gas bubbles. Additionally or alternatively, said gas outlet ports 12a or some of said gas outlet ports 12a may point transverse to and/or in the rising direction of the gas bubbles.

Moreover, as can also be seen in Fig. 2, in one adjusting position of the closing element 15 it releases other gas outlet ports 12a, 12b than in another adjusting position of the closing element 15, and/or, in one adjusting position of the closing element 15 it blocks other gas outlet ports 12a, 12b than in another adjusting position of the closing element 15. Depending on its adjusting position the closing element 15 can thus cause different gas outlet ports 12a, 12b to be released and/or blocked.

Additionally or alternatively, and as also shown in Fig. 2, in one adjusting position of the closing element 15 it releases the respective gas outlet ports 12a, 12b each to another extent than in another adjusting position of the closing element 15. Depending on its adjusting position the closing element 15 can thus cause the gas outlet ports 12a, 12b to be released to different extents, that is with different sizes of the flowable cross sections.

It is also conceivable, in a different embodiment (not shown), that in one adjusting position of the closing element 15 it releases discharge openings 17 which are associated with one or more other aeration channels 10a, 10b than in another adjusting position of the closing element 15. Depending on its adjusting position the closing element 15 can thus cause different aeration channels 10a, 10b to be in fluid communication with the outside of the aeration device 8.

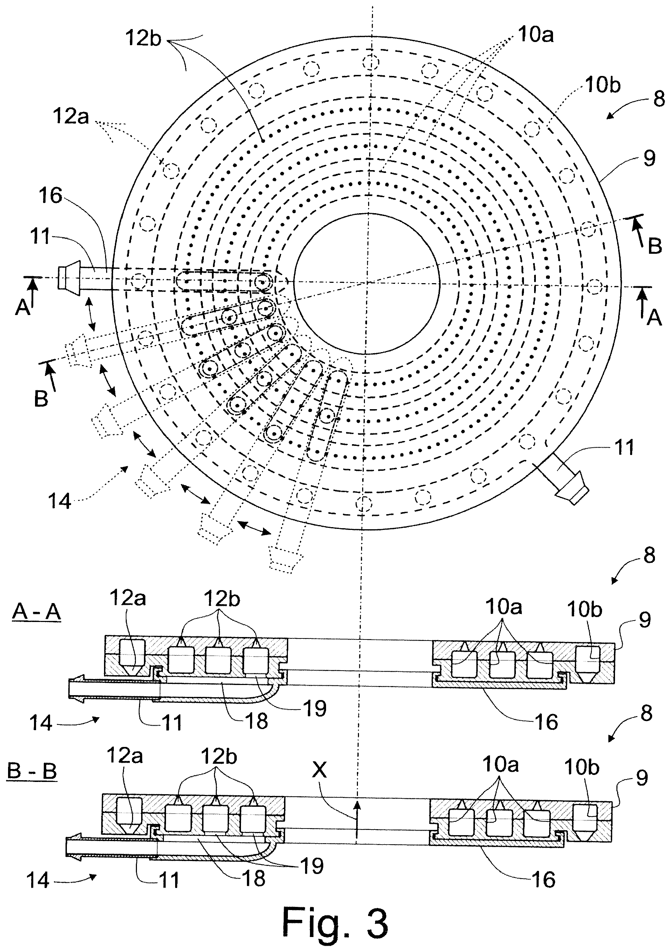

Fig. 3 shows an aeration device 8 which has, on the one hand, downward facing gas outlet ports 12a of the first type and, on the other hand, upward facing gas outlet ports 12b of only the second type. The upward facing gas outlet ports 12b are split in groups over several aeration channels 10a positioned concentrically to one another, whereby each group of gas outlet ports 12b is assigned to a respective one of these aeration channels 10a. Gas introduced into the aeration device 8 via a respective gas inlet port 11, here a common gas inlet port 11 for all of these aeration channels 10a, which is in fluid communication or can be brought into fluid communication with the aeration channels 10a, can be directed through the aeration channels 10a into the gas outlet ports 12b and from there to the outside of the aeration device 8. The gas is discharged here at the top of the aeration device 8. These aeration channels 10a and in particular the gas inlet port 11 are used to configure the aeration device 8.

The downward facing gas outlet ports 12a are assigned to a separate aeration channel 10b, which is positioned concentrically to and here radially outside of the aeration channels 10a, whereby gas introduced into the aeration device 8 via a gas inlet port 11, which is here in permanent fluid communication with this aeration channel 10b, can be directed through the aeration channel 10b into the gas outlet ports 12a of this aeration channel 10b and from there to the outside of the aeration device 8. The gas is discharged here at the bottom of the aeration device 8.

It should be noted that, in addition or alternatively to the upward facing and/or to the downward facing gas outlet ports 12a, 12b, there also may be lateral gas outlet ports 12a, 12b, i.e. with openings facing to the side (in the radial direction), which may be of the first and/or second type, these gas outlet ports 12a, 12b in an optional embodiment also being used to configure the aeration device 8.

Fig. 3 shows an exemplary embodiment, in which also, as in the embodiment in Fig. 2, the aeration device 8 has an adjusting mechanism 14, which here also is a manually operable adjusting mechanism 14. However, also in this case, instead an automatically operable adjusting mechanism 14 may be provided. Also in this embodiment the aeration device 8 is configurable with respect to a predetermined discharge of gas via the gas outlet ports 12b in that, by the adjusting mechanism 14, the discharge of gas via the gas outlet ports 12b is adjustable.

Also in this case the adjusting mechanism 14 comprises a movable closing element 16. Here the movable closing element 16 is for releasing and for blocking, at least partly, especially completely, a fluid communication of one or more gas inlet ports 11 of the housing 9 to one or more aeration channels 10a of the housing 9.

A "movable" closing element 16 means that the respective closing element 16 is movable relative to the remainder of the housing 9, in particular movable relative to the aeration channel or channels 10a, 10b and/or gas outlet ports 12a, 12b.

In this case the term "for releasing and for blocking" means that in one adjusting position of the closing element 16, when releasing a fluid communication, it allows a fluid communication of one or more gas inlet ports 11 of the housing 9 to one or more aeration channels 10a of the housing 9, and in another adjusting position if the closing element 16, when blocking a fluid communication, it does not allow a fluid communication of one or more gas inlet ports 11 of the housing 9 to one or more aeration channels 10a of a housing 9. In particular, the closing element 16 may be adjusted in such a way as to allow complete blocking and/or complete release and/or, in one or more between positions, partial release.

Additionally or alternatively the adjusting mechanism may comprise one or more control valves (not shown), in particular check valves, e.g. single use valves, each in a respective gas supply line connecting a gas source with the respective gas inlet port 11. Such control valves are for releasing and for blocking, at least partly, especially completely, a fluid communication of an assigned gas inlet port 11 of the housing 9 to one or more aeration channels 10a of the housing 9.

Also by means of the adjusting mechanism 14 shown in Fig. 3 certain characteristics of the discharged gas can be set or repeatedly adjusted, e.g. the discharge velocity of the gas, the discharge locations of the gas, the discharge direction of the gas, the gas composition and/or the gas bubble size and/or gas bubble shape at the respective outlet opening.

In particular, such a closing element 16 prevents respective gas supply lines connecting a gas source with the respective gas inlet port 11 from filling up with the liquid biological medium if no gas is supplied into the aeration channels 10a. The closing element 16 therefore preferably blocks the fluid communication of the one or more gas inlet ports 11 of the housing 9 to the one or more aeration channels 10a of the housing 9 until the beginning of an aeration sequence in a biotechnological process, and then releases the fluid communication until the end of the aeration sequence. This is preferably done automatically via the control unit 13. In this context it is also emphasized, that also aligning discharge openings 17, here of the aeration channel 10b, downwards prevents the respective gas supply lines from filling up.

As shown in Fig. 3, the closing element 16 may form a lower cover of the housing 9 and/or may be arranged, in relation to the flow direction of the gas, upstream of the respective one or more aeration channels 10a.

The term "lower" means that in the intended installed use state the closing element 16 or cover is arranged on the side of the housing 9 facing against the rising direction of the gas bubbles.

The "respective" one or more aeration channels 10a are those aeration channels 10a, to which the fluid communication can be blocked by the closing element 16.

Additionally or alternatively, and as also shown in Fig. 3, the closing element 16 may be a rotary slider or, not shown here, a linear slider. Also a closing element in form of a flap is conceivable.

The closing element 16 here and preferably is movable around the geometrical axis X which runs parallel to the rising direction of the gas bubbles. It is also conceivable, in different embodiments (not shown), that the closing element 16 is movable along the geometrical axis X which runs parallel to the rising direction of the gas bubbles, and/or that the closing element 16 is movable around or along a geometrical axis which runs transversely to the rising direction of the gas bubbles.

Here and preferably, the closing element 16 shown in Fig. 3 is disk shaped.

As can be seen in Fig. 3, here and preferably the closing element 16 comprises one or more closing element openings 18 which, for releasing, at least partly, especially completely, the fluid communication of one or more gas inlet ports 11 of the housing 9 to one or more aeration channels 10a of the housing 9, can each be brought at least in partial, especially in complete, overlap with at least one inlet opening 19 of at least one aeration channel 10a. Additionally or alternatively, the one or more closing element openings 18, for completely blocking the fluid communication of one or more gas inlet ports 11 of the housing 9 to one or more aeration channels 10a of the housing 9, can each be positioned completely offset to at least one inlet opening 19 of at least one aeration channel 10a.

Here and preferably, the closing element 16 is arranged to release and to block, at least partly, especially completely, a fluid communication to one or more, but not all, aeration channels 10a, 10b of the housing 9, and to not affect a fluid communication to one or more other aeration channels 10b, here one single other aeration channel 10b, of the housing 9. Moreover, additionally or alternatively, the closing element 16 is arranged to release and to block, at least partly, especially completely, a fluid communication of one or more, but not all, gas inlet ports 11 of the housing 9 to one or more aeration channels 10a, 10b of the housing 9, and to not affect a fluid communication of one or more other gas inlet ports 11 of the housing 9 to one or more aeration channels 10b, here one single other aeration channel 10b, of the housing 9.

According to the exemplary embodiment in Fig. 3 it is provided that the one or more aeration channels 10a, the fluid communication to which can be released and blocked by the closing element 16, are in fluid communication with and/or can be brought into fluid communication with only one type of gas outlet ports 12a, 12b, preferably only the second type of gas outlet ports 12b.

However, it is also conceivable, in a different embodiment (not shown), that the one or more aeration channels 10a, the fluid communication to which can be released and blocked by the closing element 16, are in fluid communication with and/or can be brought into fluid communication with both the first type and the second type of gas outlet ports 12a, 12b.

Here and preferably, the gas outlet ports 12b of the one or more aeration channels 10a, the fluid communication to which can be released and blocked by the closing element 16, point in the rising direction of the gas bubbles. Additionally or alternatively, said gas outlet ports 12b or some of said gas outlet ports 12b may point transverse to and/or against the rising direction of the gas bubbles.

Additionally or alternatively, and as also shown in Fig. 3, the one or more other aeration channels 10b, here the single other aeration channel 10b, the fluid communication to which cannot be affected by the closing element 16, are/is in permanent fluid communication with only one type of gas outlet ports 12a, 12b, here and preferably only the first type of gas outlet ports 12a.

Here and preferably, the gas outlet ports 12a assigned to the one or more other aeration channels 10b, here the single other aeration channel 10b, point in a different, in particular opposite, direction than the gas outlet ports 12b assigned to the aeration channel or channels 10a, the fluid communication to which can be released and blocked by the closing element 16. More preferably, these gas outlet ports 12a assigned to the one or more other aeration channels 10b, here the single other aeration channel 10b, point against the rising direction of the gas bubbles. Additionally or alternatively, said gas outlet ports 12a or some of said gas outlet ports 12a may point transverse to and/or in the rising direction of the gas bubbles.

Moreover, as can also be seen in Fig. 3, in one adjusting position of the closing element 16 it releases other inlet openings 19 than in another adjusting position of the closing element 16, and/or, in one adjusting position of the closing element 16 it blocks other inlet openings 19 than in another adjusting position of the closing element 16. Depending on its adjusting position the closing element 16 can thus cause different inlet openings 19 to be released and/or blocked and accordingly can thus cause different aeration channels 10a to be in fluid communication with a gas inlet port 11.

Additionally or alternatively, in one adjusting position of the closing element 16 it releases the respective inlet openings 19 each to another extent than in another adjusting position of the closing element 16. Depending on its adjusting position the closing element 16 can thus cause the inlet openings 19 of one or more aeration channels 10a to be released to different extents, that is with different sizes of the flowable cross sections.

Moreover, according to the exemplary embodiment in Fig. 3 it is provided that the gas inlet ports 11, from which a fluid communication to one or more aeration channels 10a of the housing 9 can be released and blocked, are arranged, here and preferably integrally, on the closing element 16 and are movable with it relative to the aeration channel or channels 10a and/or inlet openings 19 of the aeration channels 10a.

As explained above, in both exemplary embodiments of Figs. 2 and 3 the adjusting mechanism 14 may also be an automatically operable adjusting mechanism. In such a case it is conceivable that for configuration of the aeration device 8 with respect to a predetermined discharge of gas via the gas outlet ports 12a, 12b, the adjusting mechanism 14 is configured to be controlled by an electronic control unit 13 of the bioprocessing installation 1 provided for at least partial control of the bioprocessing installation 1. An example of such a control unit 13 is shown in Fig. 1, wherein pneumatic and/or hydraulic and/or electric lines may be provided for the control of the process.

Fig. 4 also shows an aeration device 8 which has, on the one hand, downward facing gas outlet ports 12a of the first type and, on the other hand, upward facing gas outlet ports 12b of only the second type. The upward facing gas outlet ports 12b are split in groups over several aeration channels 10a, each aeration channel 10a formed as a circumferential segment in the housing 9. Here four aeration channels 10a are arranged one after the other in circumferential direction, whereby each group of gas outlet ports 12b is assigned to a respective one of these aeration channels 10a. Gas introduced into the aeration device 8 via respective gas inlet ports 11, which each are in fluid communication or can be brought into fluid communication with the respective aeration channel 10a, can be directed through the aeration channels 10a into the gas outlet ports 12b and from there to the outside of the aeration device 8. Here one gas inlet port 11 is assigned to a respective one of these aeration channels 10a. The gas is discharged here at the top of the aeration device 8. These aeration channels 10a are used to configure the aeration device 8.

The downward facing gas outlet ports 12a are assigned to a separate aeration channel 10b, which is positioned concentrically to and here radially outside of the aeration channels 10a, whereby gas introduced into the aeration device 8 via a gas inlet port 11, which is here in permanent fluid communication with this aeration channel 10b, can be directed through the aeration channel 10b into the gas outlet ports 12a of this aeration channel 10b and from there to the outside of the aeration device 8. The gas is discharged here at the bottom of the aeration device 8.

It should be noted that, in addition or alternatively to the upward facing and/or to the downward facing gas outlet ports 12a, 12b, there also may be lateral gas outlet ports 12a, 12b, i.e. with openings facing to the side (in the radial direction), which may be of the first and/or second type, these gas outlet ports 12a, 12b in an optional embodiment also being used to configure the aeration device 8.

Fig. 4 shows an exemplary embodiment, in which the aeration device 8 is configurable in that at least one housing part 20a, 20b, 20c, 20d of the housing 9, which has properties influencing the discharge of gas via the gas outlet ports 12b, is detachably connected to the remainder of the housing 9 in such a way that the housing part 20a, 20b, 20c, 20d can be replaced by another housing part 20a, 20b, 20c, 20d, which has different properties influencing the discharge of gas via the gas outlet ports 12b. It is to be noted, that for schematic purpose, Fig. 4 shows each housing part 20a, 20b, 20c, 20d with a different size and/or arrangement of the gas outlet ports 12b. This is only to show that there may be different designs of housing parts 20a, 20b, 20c, 20d of this (interchangeable) type from which to choose for the configuration of the aeration device 8. However, it is also conceivable that all housing parts 20a, 20b, 20c, 20d which are attached to the remainder of the housing 9 are of the same design, i.e. have the same size and/or arrangement of the gas outlet ports 12b. It is also to be noted that additionally or alternatively to the gas outlet ports 12b of the second type, one or more of the housing parts 20a, 20b, 20c, 20d may also comprise gas outlet ports 12a of the first type.

It should be noted that in Fig. 4, as in the other exemplary embodiments, the housing 9 has a circular outer contour. However, in principle it is also possible that the outer contour has a different shape, for example oval, square, cloverleaf-shaped, star-shaped or the like. In the case of a cloverleaf shape, for example, it is conceivable that in each leaf of the cloverleaf a different one of the housing parts 20a, 20b, 20c, 20d and/or types of discharge openings 12a, 12b is arranged.

The different designs, in particular the different sizes and/or arrangements of gas outlet ports 12a, 12b, lead to different properties influencing the discharge of gas via the gas outlet ports 12a, 12b. Due to said properties of the housing parts 20a, 20b, 20c, 20d certain characteristics of the discharged gas can be set, e.g. the discharge velocity of the gas, the discharge locations of the gas, the discharge direction of the gas and/or the gas bubble size and/or gas bubble shape at the respective discharge opening.

The term "detachably connected" means that the respective housing part 20a, 20b, 20c, 20d is non-destructively detachable to the remainder of the housing 9.

Thereby, the respective housing part 20a, 20b, 20c, 20d can be replaced, such that a non-destructive removal and replacement is possible. In particular the aeration device 8 comprises a set of several functionally identical housing parts of this interchangeable type, each of which can be mounted to the remainder of the housing 9 according to a specific biotechnological process and/or specific stage within a biotechnological process.

Here and preferably, the at least one housing part 20a, 20b, 20c, 20d is detachably connected to the remainder of the housing 9 by a positive connection (form-fit connection) and/or non-positive connection (force-locking connection), in particular a snap connection or plug connection.

As can be seen in Fig. 4, here and preferably the at least one housing part 20a, 20b, 20c, 20d which is detachably connected to the remainder of the housing 9, forms one or more gas outlet ports 12a, 12b of the housing 9 and/or at least a section of one or more aeration channels 10a of the housing 9, preferably a section defining at least the upper boundary and optionally one or both lateral boundaries of the respective aeration channel 10a.

Furthermore, according to the exemplary embodiment in Fig. 4 it is provided that one or more other aeration channels 10b of the housing 9 are formed in its entirety by the remainder of the housing 9. Said one or more other aeration channels 10b are not covered or formed by the at least one housing part 20a, 20b, 20c, 20d which is detachably connected to the remainder of the housing 9.

Moreover, here and preferably a respective one of the gas inlet ports 11 of the housing 9 is assigned to each housing part 20a, 20b, 20c, 20d which is detachably connected to the remainder of the housing 9. Here and preferably, the respective gas inlet port 11 of the housing 9, preferably each gas inlet port 11 of the housing 9, is arranged, preferably integrally, on the remainder of the housing 9.

As shown in Fig. 4, the at least one housing part 20a, 20b, 20c, 20d forms an upper part of the housing 9 and is arranged, in relation to the flow direction of the gas, downstream of the respective one or more aeration channels 10a and/or inlet openings 19 of the one or more aeration channels 10a.

The term "upper" means that in the intended installed use state the housing part 20a, 20b, 20c, 20d is arranged on the side of the housing 9 facing in the rising direction of the gas bubbles, that is in the vertical direction and parallel to direction of gravity.

The "respective" one or more aeration channels 10a are those aeration channels 10a, the fluid communication of which to the outside of the aeration device 8 is established via the gas outlet ports 12a, 12b of the housing part 20a, 20b, 20c, 20d.

As also can be seen in Fig. 4, here and preferably the at least one housing part 20a, 20b, 20c, 20d establishes a permanent fluid communication of the respective aeration channel 10a to the outside of the aeration device 8.

Here and preferably, the gas outlet ports 12a, 12b formed by the at least one housing part 20a, 20b, 20c, 20d are gas outlet ports 12a, 12b of both the first type and the second type of gas outlet ports 12a, 12b. However, it is also conceivable that the gas outlet ports 12a, 12b are of only one type of gas outlet ports 12a, 12b, preferably only the second type of gas outlet ports 12b. The gas outlet ports 12a, 12b formed by the at least one housing part 20a, 20b, 20c, 20d here and preferably point in, but may additionally or alternatively also point transverse to and/or against the rising direction of the gas bubbles.

Additionally or alternatively, and as also shown in Fig. 4, one or more other aeration channels 10b, here one single other aeration channel 10b, formed in its entirety by the remainder of the housing 9, are/is in permanent fluid communication with only one type of gas outlet ports 12a, 12b, preferably only the first type of gas outlet ports 12a.

Here and preferably, the gas outlet ports 12a assigned to the one or more other aeration channels 10b, here the single other aeration channel 10b, point in a different, in particular opposite, direction than the gas outlet ports 12a, 12b formed by the at least one housing part 20a-20d. More preferably, the gas outlet ports 12a assigned to the one or more other aeration channels 10b, here the single other aeration channel 10b, point against the rising direction of the gas bubbles. Additionally or alternatively, said gas outlet ports 12a or some of said gas outlet ports 12a may point transverse to and/or in the rising direction of the gas bubbles.

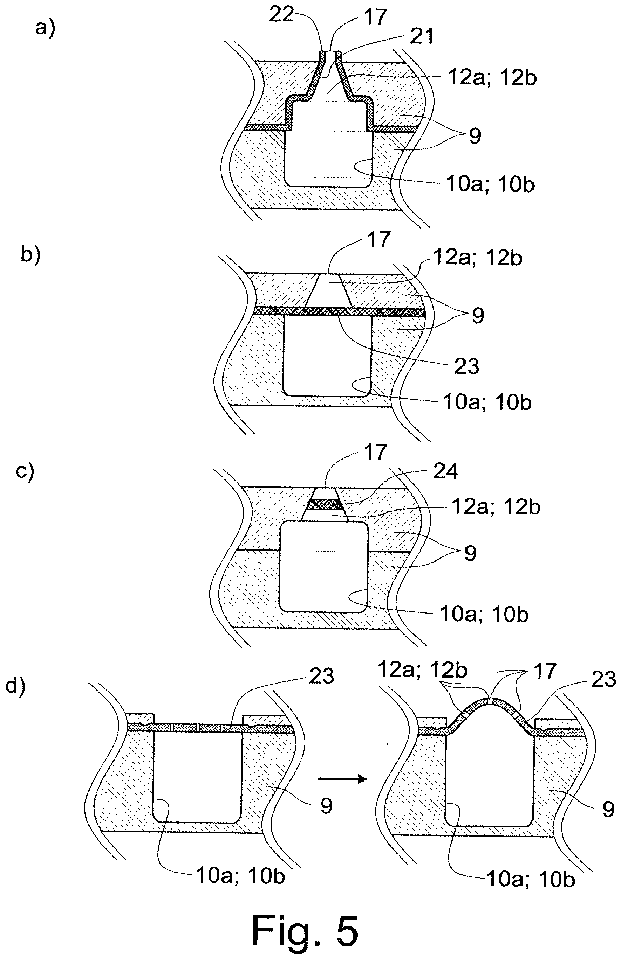

Fig. 5 shows different examples of the design of the gas outlet ports 12a, 12b of the proposed aeration device 8 and/or of the transition between the respective aeration channel 10a or the aeration channel 10b and the assigned gas outlet ports 12a, 12b.

These preferred embodiments of aeration channels 10a, 10b with gas outlet ports 12a, 12b, which are described below, may be provided in the aeration device 8 described above. However, these can also be used advantageously on their own in an aeration device 8 according to an independent further teaching. All explanations given for the above aeration device 8 according to the first teaching are therefore fully applicable to each further teaching.

Fig. 5a) shows an exemplary embodiment according to an independent second teaching with an aeration device 8 for a bioprocessing installation 1, in particular a bioreactor 2, comprising a housing 9, wherein the housing 9 comprises one or more aeration channels 10a, 10b in its interior, wherein the housing 9 further comprises one or more gas inlet ports 11, via each of which a gas can be introduced into at least one aeration channel 10a, 10b of the housing 9, and wherein the housing 9 further comprises a plurality of gas outlet ports 12a, 12b, via each of which the gas can be discharged from the respective aeration channel 10a, 10b to the outside of the aeration device 8. According to this independent second teaching the surface on the inside of one or more gas outlet ports 12a, 12b of the housing 9 and/or of at least a section of one or more aeration channels 10a, 10b of the housing 9, preferably of a section defining at least the upper boundary and optionally one or both lateral boundaries of the respective aeration channel 10a, 10b, is provided, at least partly, especially completely, with a coating or insert 21. A suitable coating or insert 21 may prevent the respective gas outlet port 12a, 12b from clogging.

Here and preferably, the coating or insert 21 is made of a hydrophobic material, more preferably a silicone material. According to the exemplary embodiment in Fig. 5a) the coating or insert 21 protrudes from the housing 9 to form a projection 22, preferably a cylindrical, conical or funnel-shaped projection 22.

Additionally or alternatively, the coating or insert 21 may be made of flexible material. In particular the protruding part of the coating or insert 21 may be made of flexible material.

It is also conceivable, in a different embodiment (not shown), that the surface on the inside of one or more gas outlet ports 12a, 12b of the housing 9 and/or of at least a section of one or more aeration channels 10a, 10b of the housing 9, preferably of a section defining at least the upper boundary and optionally one or both lateral boundaries of the respective aeration channel 10a, 10b, is provided partly with a coating or an insert of a hydrophilic material.

Figs. 5b) and c) show exemplary embodiments according to an independent third teaching with an aeration device 8 for a bioprocessing installation 1, in particular a bioreactor 2, comprising a housing 9, wherein the housing 9 comprises one or more aeration channels 10a, 10b in its interior, wherein the housing 9 further comprises one or more gas inlet ports 11, via each of which a gas can be introduced into at least one aeration channel 10a, 10b of the housing 9, and wherein the housing 9 further comprises a plurality of gas outlet ports 12a, 12b, via each of which the gas can be discharged from the respective aeration channel 10a, 10b to the outside of the aeration device 8. According to this independent third teaching, for one or more aeration channels 10a, 10b of the housing 9 a membrane 23 and/or a filter material 24, preferably made of a hydrophobic material, is arranged between the respective aeration channel 10a, 10b and one or more gas outlet ports 12a, 12b of the housing 9 (Fig. 5b)), such that the gas can be discharged from the respective aeration channel 10a, 10b through the membrane 23 and/or filter material 24 to the outside of the aeration device 8. Such a membrane 23 and/or filter material 24 is for example also provided on housing parts 20a, 20b in Fig. 4. Such a hydrophobic membrane 23 and/or filter material 24 in particular functions as check valve and may prevent the housing 9 and respective gas supply lines connecting a gas source with the respective gas inlet port 11 from filling up with the liquid biological medium if no gas exits through the discharge openings 17 of the gas outlet ports 12a, 12b. Moreover, a suitable filter material 24, e.g. a sintered material, can be used for sterile filtration of gas, especially air, through the aeration device 8. Such a device could be an aeration device 8 and an inlet air sterile filter at the same time.

Additionally or alternatively, such a membrane 23 and/or a filter material 24, preferably made of a hydrophobic material, is arranged inside one or more gas outlet ports 12a, 12b of the housing 9 (Fig. 5c)), such that the gas can be discharged from the respective aeration channel 10a, 10b through the membrane 23 and/or filter material 24 to the outside of the aeration device 8.

Fig. 5d) shows an exemplary embodiment according to an independent fourth teaching with an aeration device for a bioprocessing installation 1, in particular a bioreactor 2, comprising a housing 9, wherein the housing 9 comprises one or more aeration channels 10a, 10b in its interior, wherein the housing 9 further comprises one or more gas inlet ports 11, via each of which a gas can be introduced into at least one aeration channel 10a, 10b of the housing 9, and wherein the housing 9 further comprises a plurality of gas outlet ports 12a, 12b, via each of which the gas can be discharged from the respective aeration channel 10a, 10b to the outside of the aeration device 8. According to this independent fourth teaching one or more aeration channels 10a, 10b of the housing 9 are bordered at the top by a membrane 23 made of a flexible material, preferably a silicone material, wherein the membrane 23 forms one or more gas outlet ports 12a, 12b of the housing 9, wherein preferably, due to the flexibility of the material of the membrane 23, the size of the cross-sectional area of the gas outlet ports 12a, 12b formed by the membrane 23 changes depending on the gas pressure in the respective aeration channel 10a, 10b. Such a flexible membrane 23 may prevent the housing 9 and respective gas supply lines connecting a gas source with the respective gas inlet port 11 from filling up with the liquid biological medium if no gas exits through the discharge openings 17 of the gas outlet ports 12a, 12b. Furthermore, such a flexible membrane 23 may prevent the respective gas outlet port 12a, 12b formed by the membrane 23 from clogging.

More preferably, as can be seen in Fig. 5d), the size of the cross-sectional area of the gas outlet ports 12a, 12b formed by the membrane 23 is smaller, when the gas pressure in the respective aeration channel 10a, 10b is low, and is larger, when the gas pressure in the respective aeration channel 10a, 10b is high. The terms "low" and "high" should be understood relative to each other, which means that the size of the cross-sectional area of the gas outlet ports 12a, 12b formed by the membrane 23 increases with the gas pressure.

More preferably, one or more of the gas outlet ports 12a, 12b formed by the membrane 23 are closed, when the gas pressure in the respective aeration channel 10a, 10b is low, and are opened, when the gas pressure in the respective aeration channel 10a, 10b is high. This means that with no gas pressure and preferably below a certain gas pressure, at least some gas outlet ports 12a, 12b are closed. In particular, thereby it is possible to change the amount of active (released) gas outlet ports 12a, 12b. At higher gas pressure more gas outlet ports 12a, 12b may be active than at low gas pressure.

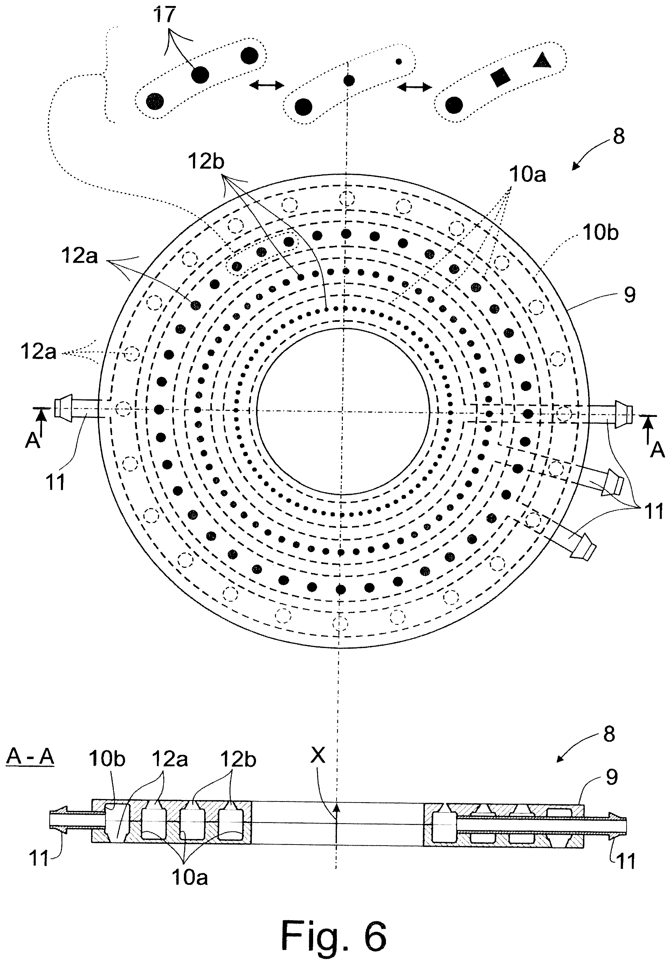

Fig. 6 shows an aeration device 8 according to a fifth teaching, which has, on the one hand, downward facing gas outlet ports 12a of the first type and, on the other hand, as in Fig. 2 upward facing gas outlet ports 12a, 12b of the first type and the second type. The upward facing gas outlet ports 12a, 12b are split in groups over several aeration channels 10a positioned concentrically to one another, whereby each group of gas outlet ports 12a, 12b is assigned to a respective one of these aeration channels 10a. Gas introduced into the aeration device 8 via respective gas inlet ports 11, which each are in fluid communication or can be brought into fluid communication with the respective aeration channel 10a, can be directed through the aeration channels 10a into the gas outlet ports 12a, 12b and from there to the outside of the aeration device 8. Here one gas inlet port 11 is assigned to a respective one of these aeration channels 10a. The gas is discharged here at the top of the aeration device 8. These aeration channels 10a and in particular the gas inlet ports 11 are used to configure the aeration device 8, here by electronically controlled check valves, which are connected upstream of the gas inlet ports 11 in the direction of flow. The check valves (not shown) can be controlled or regulated via the electronic control unit 13.

The downward facing gas outlet ports 12a are assigned to a separate aeration channel 10b, which is positioned concentrically to and here radially outside of the aeration channels 10a, whereby gas introduced into the aeration device 8 via a gas inlet port 11, which is here in permanent fluid communication with this aeration channel 10b, can be directed through the aeration channel 10b into the gas outlet ports 12a of this aeration channel 10b and from there to the outside of the aeration device 8. The gas is discharged here at the bottom of the aeration device 8.

It should be noted that, in addition or alternatively to the upward facing and/or to the downward facing gas outlet ports 12a, 12b, there also may be lateral gas outlet ports 12a, 12b, i.e. with discharge openings facing to the side (in the radial direction), which may be of the first and/or second type, these gas outlet ports 12a, 12b in an optional embodiment also being used to configure the aeration device 8.

Fig. 6 shows an exemplary embodiment according to an independent fifth teaching with an aeration device 8 for a bioprocessing installation 1, in particular a bioreactor 2, comprising a housing 9, wherein the housing 9 comprises one or more aeration channels 10a, 10b in its interior, wherein the housing 9 further comprises one or more gas inlet ports 11, via each of which a gas can be introduced into at least one aeration channel 10a, 10b of the housing 9, and wherein the housing 9 further comprises a plurality of gas outlet ports 12a, 12b, via each of which the gas can be discharged from the respective aeration channel 10a, 10b to the outside of the aeration device 8. According to this independent fifth teaching at least some, in particular all, of the gas outlet ports 12a, 12b of a respective one of the aeration channels 10a, 10b are non-uniform, or at least one group of gas outlet ports 12a, 12b of a respective one of the aeration channels 10a, 10b is non-uniform.

The term "non-uniform" means on one hand that some, in particular all, of the gas outlet ports 12a, 12b may each have, in cross-section and/or in longitudinal section, a non-uniform outer contour, e.g. a triangular or square outer contour, and on the other hand that within one group defined by a plurality of gas outlet ports 12a, 12b, in cross-section and/or in longitudinal section, not all gas outlet ports 12a, 12b have the same outer contour. A "group" of gas outlet ports 12a, 12b is defined as all gas outlet ports 12a, 12b that are assigned to the same aeration channel 10a, 10b. A "group" of gas outlet ports 12a, 12b is, additionally or alternatively, defined as all gas outlet ports 12a, 12b of the same type.

Preferably, as symbolized in Fig. 6, the size of the cross-sectional area of the gas outlet ports 12a, 12b of at least one, in particular each, of the aeration channels 10a, 10b varies along the aeration channel 10a, 10b, preferably increases from the gas inlet port 11 along the aeration channel 10a, 10b. The latter ensures, for example, that nearly the same gas pressure is present at each discharge opening 17 in that row of gas outlet ports 12a, 12b.

Additionally or alternatively, as also symbolized in Fig. 6, the shape of the cross-sectional area of at least some, in particular all, of the gas outlet ports 12a, 12b of at least one, in particular all, of the aeration channels 10a, 10b is not circular.

According to an independent sixth teaching a bioprocessing installation 1, in particular a bioreactor 2, is provided, comprising a container 3, preferably a dimensionally stable vessel or flexible bag 4, in which a liquid biological medium consisting of the substances intended for a biotechnological process can be accommodated, wherein the bioprocessing installation 1 further comprises a proposed aeration device 8. All explanations given for the aeration device 8 according to the first to fifth teaching are fully applicable to this teaching.

## Claims

1. An aeration device for a bioprocessing installation (1), in particular a bioreactor (2), comprising a housing (9),
wherein the housing (9) comprises one or more aeration channels (10a, 10b) in its interior,
wherein the housing (9) further comprises one or more gas inlet ports (11), via each of which a gas can be introduced into at least one aeration channel (10a, 10b) of the housing (9), and
wherein the housing (9) further comprises a plurality of gas outlet ports (12a, 12b), via each of which the gas can be discharged from the respective aeration channel (10a, 10b) to the outside of the aeration device (8),
**characterized in that**
the aeration device (8) is configurable with respect to a predetermined discharge of gas via the gas outlet ports (12a, 12b).

2. The aeration device according to claim 1, **characterized in that** the gas outlet ports (12a, 12b) are provided in the form of a first type of gas outlet ports (12a) which consists of gas outlet ports (12a) having an discharge opening (17) with a diameter larger than 0,2 mm, preferably of 0,25 to 4 mm, more preferably of 0,5 to 1,0 mm, and/or a cross-sectional area larger than 0,03 mm², preferably of 0,05 to 12,0 mm², more preferably of 0,2 to 0,8 mm², and/or in the form of a second type of gas outlet ports (12b) which consists of gas outlet ports (12b) having an discharge opening (17) with a diameter of at most 0,2 mm, preferably of 0,005 to 0,2 mm, more preferably of 0,02 to 0,18 mm, and/or a cross-sectional area of at most 0,03 mm², preferably of 0,00002 to 0,03 mm², more preferably of 0,0003 to 0,025 mm².

3. The aeration device according to claim 1 or 2, **characterized in that** the aeration device (8) has an adjusting mechanism (14), in particular a manually or automatically operable adjusting mechanism (14), and is configurable with respect to a predetermined discharge of gas via the gas outlet ports (12a, 12b) **in that**, by the adjusting mechanism (14), the discharge of gas via the gas outlet ports (12a, 12b) is adjustable.

4. The aeration device according to claim 3, **characterized in that** the adjusting mechanism (14) comprises a movable closing element (15) for releasing and for blocking, at least partly, especially completely, a fluid communication of one or more aeration channels (10a) of the housing (9) to the outside of the aeration device (8).

5. The aeration device according to claim 4, **characterized in that** the closing element (15) forms an upper cover of the housing (9) and/or is arranged, in relation to the flow direction of the gas, downstream of the respective one or more aeration channels (10a) and/or gas outlet ports (12a, 12b), and/or, **in that** the closing element (15) is a rotary slider or linear slider, and/or, **in that** the closing element (15) is movable around or along a geometrical axis (X) which runs parallel or transversely to the rising direction of the gas bubbles, and/or, **in that** the closing element (15) is disk shaped.

6. The aeration device according to claim 4 or 5, **characterized in that** the closing element (15) comprises one or more closing element openings (18) which, for releasing, at least partly, especially completely, the fluid communication of one or more aeration channels (10a) of the housing (9) to the outside of the aeration device (8), can each be brought at least in partial, especially in complete, overlap with at least one discharge opening (17), and/or, which, for completely blocking the fluid communication of one or more aeration channels (10a) of the housing (9) to the outside of the aeration device (8), can each be positioned completely offset to at least one discharge opening (17).

7. The aeration device according to one of claims 4 to 6, **characterized in that** the closing element (15) is arranged to release and to block, at least partly, especially completely, a fluid communication of one or more, but not all, aeration channels (10a, 10b) of the housing (9) to the outside of the aeration device (8), and to not affect a fluid communication of one or more other aeration channels (10b) of the housing (9) to the outside of the aeration device (8).

8. The aeration device according to claim 7, **characterized in that** the one or more aeration channels (10a), the fluid communication of which to the outside of the aeration device (8) can be released and blocked by the closing element (15), are in fluid communication with and/or can be brought into fluid communication with only one type of gas outlet ports (12a, 12b), preferably only the second type of gas outlet ports (12b), or with both the first type and the second type of gas outlet ports (12a, 12b), preferably **in that** these gas outlet ports (12a, 12b) point in and/or transverse to and/or against the rising direction of the gas bubbles,
and/or, **in that** the one or more other aeration channels (10b), the fluid communication of which to the outside of the aeration device (8) cannot be affected by the closing element (15), are in permanent fluid communication with only one type of gas outlet ports (12a, 12b), preferably only the first type of gas outlet ports (12a), preferably **in that** these gas outlet ports (12a, 12b) assigned to the one or more other aeration channels (10b) point in a different, in particular opposite, direction than the gas outlet ports (12a, 12b) assigned to the aeration channel or channels (10a), the fluid communication of which to the outside of the aeration device (8) can be released and blocked by the closing element (15), more preferably **in that** these gas outlet ports (12a, 12b) assigned to the one or more other aeration channels (10b) point against and/or transverse to and/or in the rising direction of the gas bubbles.

9. The aeration device according to one of claims 4 to 8, **characterized in that** in one adjusting position of the closing element (15) it releases other gas outlet ports (12a, 12b) than in another adjusting position of the closing element (15), and/or, **in that** in one adjusting position of the closing element (15) it blocks other gas outlet ports (12a, 12b) than in another adjusting position of the closing element (15).

10. The aeration device according to one of claims 4 to 9, **characterized in that** in one adjusting position of the closing element (15) it releases the respective gas outlet ports (12a, 12b) each to another extent than in another adjusting position of the closing element (15).

11. The aeration device according to one of claims 4 to 10, **characterized in that** in one adjusting position of the closing element (15), it releases discharge openings (17) which are associated with one or more other aeration channels (10a, 10b) than in another adjusting position of the closing element (15).

12. The aeration device according to one of claims 3 to 11, **characterized in that** the adjusting mechanism (14) comprises a movable closing element (16) for releasing and for blocking, at least partly, especially completely, a fluid communication of one or more gas inlet ports (11) of the housing (9) to one or more aeration channels (10a) of the housing (9).

13. The aeration device according to claim 12, **characterized in that** the closing element (16) forms a lower cover of the housing (9) and/or is arranged, in relation to the flow direction of the gas, upstream of the respective one or more aeration channels (10a), and/or, **in that** the closing element (16) is a rotary slider or linear slider, and/or, **in that** the closing element (16) is movable around or along a geometrical axis (X) which runs parallel or transversely to the rising direction of the gas bubbles, and/or, **in that** the closing element (16) is disk shaped.

14. The aeration device according to claim 12 or 13, **characterized in that** the closing element (16) comprises one or more closing element openings (18) which, for releasing, at least partly, especially completely, the fluid communication of one or more gas inlet ports (11) of the housing (9) to one or more aeration channels (10a) of the housing (9), can each be brought at least in partial, especially in complete, overlap with at least one inlet opening (19) of at least one aeration channel (10a), and/or, which, for completely blocking the fluid communication of one or more gas inlet ports (11) of the housing (9) to one or more aeration channels (10a) of the housing (9), can each be positioned completely offset to at least one inlet opening (19) of at least one aeration channel (10a).

15. The aeration device according to one of claims 12 to 14, **characterized in that** the closing element (16) is arranged to release and to block, at least partly, especially completely, a fluid communication to one or more, but not all, aeration channels (10a, 10b) of the housing (9), and to not affect a fluid communication to one or more other aeration channels (10b) of the housing (9), and/or, **in that** the closing element (16) is arranged to release and to block, at least partly, especially completely, a fluid communication of one or more, but not all, gas inlet ports (11) of the housing (9) to one or more aeration channels (10a, 10b) of the housing (9), and to not affect a fluid communication of one or more other gas inlet ports (11) of the housing (9) to one or more aeration channels (10b) of the housing (9).

16. The aeration device according to claim 15, **characterized in that** the one or more aeration channels (10a), the fluid communication to which can be released and blocked by the closing element (16), are in fluid communication with and/or can be brought into fluid communication with only one type of gas outlet ports (12a, 12b), preferably only the second type of gas outlet ports (12b), or with both the first type and the second type of gas outlet ports (12a, 12b), preferably **in that** these gas outlet ports (12a, 12b) point in and/or transverse to and/or against the rising direction of the gas bubbles,
and/or, **in that** the one or more other aeration channels (10b), the fluid communication to which cannot be affected by the closing element (16), are in permanent fluid communication with only one type of gas outlet ports (12a, 12b), preferably only the first type of gas outlet ports (12a), preferably **in that** these gas outlet ports (12a, 12b) assigned to the one or more other aeration channels (10b) point in a different, in particular opposite, direction than the gas outlet ports (12a, 12b) assigned to the aeration channel or channels (10a), the fluid communication to which can be released and blocked by the closing element (16), more preferably **in that** these gas outlet ports (12a, 12b) assigned to the one or more other aeration channels (10b) point against and/or transverse to and/or in the rising direction of the gas bubbles.

17. The aeration device according to one of claims 12 to 16, **characterized in that** in one adjusting position of the closing element (16) it releases other inlet openings (19) than in another adjusting position of the closing element (16), and/or, **in that** in one adjusting position of the closing element (16) it blocks other inlet openings (19) than in another adjusting position of the closing element (16).

18. The aeration device according to one of claims 12 to 17, **characterized in that** in one adjusting position of the closing element (16) it releases the respective inlet openings (19) each to another extent than in another adjusting position of the closing element (16).

19. The aeration device according to one of claims 12 to 18, **characterized in that** the gas inlet ports (11), from which a fluid communication to one or more aeration channels (10a) of the housing (9) can be released and blocked, are arranged, preferably integrally, on the closing element (16) and are movable with it relative to the aeration channel or channels (10a) and/or inlet openings (19) of the aeration channel or channels (10a).

20. The aeration device according to one of claims 3 to 19, **characterized in that** for configuration of the aeration device (8) with respect to a predetermined discharge of gas via the gas outlet ports (12a, 12b), the adjusting mechanism (14) is configured to be controlled by an electronic control unit (13) of the bioprocessing installation (1) provided for at least partial control of the bioprocessing installation (1).

21. The aeration device according to one of the preceding claims, **characterized in that** the aeration device (8) is configurable **in that** at least one housing part (20a, 20b, 20c, 20d) of the housing (9), which has properties influencing the discharge of gas via the gas outlet ports (12a, 12b), is detachably connected to the remainder of the housing (9) in such a way that the housing part (20a, 20b, 20c, 20d) can be replaced by another housing part (20a, 20b, 20c, 20d) which has different properties influencing the discharge of gas via the gas outlet ports (12a, 12b).

22. The aeration device according to claim 21, **characterized in that** the at least one housing part (20a, 20b, 20c, 20d) is detachably connected to the remainder of the housing (9) by a positive connection and/or non-positive connection, in particular a snap connection or plug connection.

23. The aeration device according to claim 21 or 22, **characterized in that** the at least one housing part (20a, 20b, 20c, 20d) which is detachably connected to the remainder of the housing (9), forms one or more gas outlet ports (12a, 12b) of the housing (9) and/or at least a section of one or more aeration channels (10a) of the housing (9), preferably a section defining at least the upper boundary and optionally one or both lateral boundaries of the respective aeration channel (10a).

24. The aeration device according to claim 23, **characterized in that** one or more other aeration channels (10b) of the housing (9) are formed in its entirety by the remainder of the housing (9).

25. The aeration device according to one of claims 21 to 24, **characterized in that** a respective one of the gas inlet ports (11) of the housing (9) is assigned to each housing part (20a, 20b, 20c, 20d) which is detachably connected to the remainder of the housing (9), preferably **in that** the respective gas inlet port (11) of the housing (9), preferably each gas inlet port (11) of the housing (9), is arranged, preferably integrally, on the remainder of the housing (9).

26. The aeration device according to one of claims 21 to 25, **characterized in that** the at least one housing part (20a, 20b, 20c, 20d) forms an upper part of the housing (9) and is arranged, in relation to the flow direction of the gas, downstream of the respective one or more aeration channels (10a) and/or inlet openings (19) of the one or more aeration channels (10a).

27. The aeration device according to one of claims 21 to 26, **characterized in that** the at least one housing part (20a, 20b, 20c, 20d) establishes a permanent fluid communication of the respective aeration channel (10a) to the outside of the aeration device (8).

28. The aeration device according to one of claims 23 to 27, **characterized in that** the gas outlet ports (12a, 12b) formed by the at least one housing part (20a, 20b, 20c, 20d) are gas outlet ports (12a, 12b) of only one type of gas outlet ports (12a, 12b), preferably only the second type of gas outlet ports (12b), or of both the first type and the second type of gas outlet ports (12a, 12b), preferably **in that** these gas outlet ports (12a, 12b) point in and/or transverse to and/or against the rising direction of the gas bubbles,
and/or, **in that** the one or more other aeration channels (10b) which are formed in its entirety by the remainder of the housing (9), are in permanent fluid communication with only one type of gas outlet ports (12a, 12b), preferably only the first type of gas outlet ports (12a), preferably **in that** these gas outlet ports (12a, 12b) assigned to the one or more other aeration channels (10b) point in a different, in particular opposite, direction than the gas outlet ports (12a, 12b) formed by the at least one housing part (20a, 20b, 20c, 20d), more preferably **in that** these gas outlet ports (12a, 12b) assigned to the one or more other aeration channels (10b) point against and/or transverse to and/or in the rising direction of the gas bubbles.

29. An aeration device for a bioprocessing installation (1), in particular a bioreactor (2), in particular the aeration device (8) according to one of the preceding claims, comprising a housing (9),
wherein the housing (9) comprises one or more aeration channels (10a, 10b) in its interior,
wherein the housing (9) further comprises one or more gas inlet ports (11), via each of which a gas can be introduced into at least one aeration channel (10a, 10b) of the housing (9), and
wherein the housing (9) further comprises a plurality of gas outlet ports (12a, 12b), via each of which the gas can be discharged from the respective aeration channel (10a, 10b) to the outside of the aeration device (8),
**characterized in that**
the surface on the inside of one or more gas outlet ports (12a, 12b) of the housing (9) and/or of at least a section of one or more aeration channels (10a, 10b) of the housing (9), preferably of a section defining at least the upper boundary and optionally one or both lateral boundaries of the respective aeration channel (10a, 10b), is provided, at least partly, especially completely, with a coating or insert (21), preferably made of a hydrophobic material, more preferably a silicone material, preferably **in that** the coating or insert (21) protrudes from the housing (9) to form a projection (22), preferably a cylindrical, conical or funnel-shaped projection (22).

30. An aeration device for a bioprocessing installation (1), in particular a bioreactor (2), in particular the aeration device (8) according to one of the preceding claims, comprising a housing (9),
wherein the housing (9) comprises one or more aeration channels (10a, 10b) in its interior,
wherein the housing (9) further comprises one or more gas inlet ports (11), via each of which a gas can be introduced into at least one aeration channel (10a, 10b) of the housing (9), and
wherein the housing (9) further comprises a plurality of gas outlet ports (12a, 12b), via each of which the gas can be discharged from the respective aeration channel (10a, 10b) to the outside of the aeration device (8),
**characterized in that**
for one or more aeration channels (10a, 10b) of the housing (9) a membrane (23) and/or a filter material (24), preferably made of a hydrophobic material, is arranged between the respective aeration channel (10a, 10b) and one or more gas outlet ports (12a, 12b) of the housing (9) and/or is arranged inside one or more gas outlet ports (12a, 12b) of the housing (9), such that the gas can be discharged from the respective aeration channel (10a, 10b) through the membrane (23) and/or filter material (24) to the outside of the aeration device (8).

31. An aeration device for a bioprocessing installation (1), in particular a bioreactor (2), in particular the aeration device (8) according to one of the preceding claims, comprising a housing (9),
wherein the housing (9) comprises one or more aeration channels (10a, 10b) in its interior,
wherein the housing (9) further comprises one or more gas inlet ports (11), via each of which a gas can be introduced into at least one aeration channel (10a, 10b) of the housing (9), and
wherein the housing (9) further comprises a plurality of gas outlet ports (12a, 12b), via each of which the gas can be discharged from the respective aeration channel (10a, 10b) to the outside of the aeration device (8),
**characterized in that**
one or more aeration channels (10a, 10b) of the housing (9) are bordered at the top by a membrane (23) made of a flexible material, preferably a silicone material, and **in that** the membrane (23) forms one or more gas outlet ports (12a, 12b) of the housing (9), preferably **in that**, due to the flexibility of the material of the membrane (23), the size of the cross-sectional area of the gas outlet ports (12a, 12b) formed by the membrane (23) changes depending on the gas pressure in the respective aeration channel (10a, 10b),
more preferably **in that** the size of the cross-sectional area of the gas outlet ports (12a, 12b) formed by the membrane (23) is smaller, when the gas pressure in the respective aeration channel (10a, 10b) is low, and is larger, when the gas pressure in the respective aeration channel (10a, 10b) is high, more preferably **in that** one or more of the gas outlet ports (12a, 12b) formed by the membrane (23) are closed, when the gas pressure in the respective aeration channel (10a, 10b) is low, and are opened, when the gas pressure in the respective aeration channel (10a, 10b) is high.

32. An aeration device for a bioprocessing installation (1), in particular a bioreactor (2), in particular the aeration device (8) according to one of the preceding claims, comprising a housing (9),
wherein the housing (9) comprises one or more aeration channels (10a, 10b) in its interior,
wherein the housing (9) further comprises one or more gas inlet ports (11), via each of which a gas can be introduced into at least one aeration channel (10a, 10b) of the housing (9), and
wherein the housing (9) further comprises a plurality of gas outlet ports (12a, 12b), via each of which the gas can be discharged from the respective aeration channel (10a, 10b) to the outside of the aeration device (8),
**characterized in that**
at least some, in particular all, of the gas outlet ports (12a, 12b) of a respective one of the aeration channels (10a, 10b) are non-uniform, or the group of gas outlet ports (12a, 12b) of a respective one of the aeration channels (10a, 10b) is non-uniform, preferably **in that** the size of the cross-sectional area of the gas outlet ports (12a, 12b) of at least one, in particular each, of the aeration channels (10a, 10b) varies along the aeration channel (10a, 10b), preferably increases from the gas inlet port (11) along the aeration channel (10a, 10b), and/or **in that** the shape of the cross-sectional area of at least some, in particular all, of the gas outlet ports (12a, 12b) of at least one, in particular all, of the aeration channels (10a, 10b) is not circular.

33. A bioprocessing installation, in particular a bioreactor (2), comprising a container (3), preferably a dimensionally stable vessel or flexible bag (4), in which a liquid biological medium consisting of the substances intended for a biotechnological process can be accommodated, further comprising an aeration device (8) according to one of the preceding claims.
